Europäisches Patentamt

European Patent Office

Office européen des brevets

(19)

(11) Veröffentlichungsnummer: **0 036 443**
**B1**

(12) **EUROPÄISCHE PATENTSCHRIFT**

(45) Veröffentlichungstag der Patentschrift: **05.03.86**

(51) Int. Cl.⁴: **A 61 B 5/14**, A 61 B 17/32

(21) Anmeldenummer: **80107078.0**

(22) Anmeldetag: **15.11.80**

(54) Blutlanzettenvorrichtung zur Entnahme von Blut für Diagnosezwecke.

(30) Priorität: **22.03.80 DE 3011211**

(43) Veröffentlichungstag der Anmeldung:
**30.09.81 Patentblatt 81/39**

(45) Bekanntmachung des Hinweises auf die
Patenterteilung:
**05.03.86 Patentblatt 86/10**

(84) Benannte Vertragsstaaten:
**AT BE CH DE FR GB IT LI LU NL SE**

(56) Entgegenhaltungen:
**DE-A-1 225 819**
**FR-A- 393 189**
**FR-A-2 365 331**
**US-A-2 442 416**
**US-A-3 030 959**
**US-A-3 656 472**

(73) Patentinhaber: **Boehringer Mannheim GmbH**
**Sandhofer Strasse 116**
**D-6800 Mannheim 31 (DE)**

(72) Erfinder: **Meinecke, Dieter**
**Meerwiesenstrasse 6**
**D-6800 Mannheim 1 (DE)**
Erfinder: **Schmidt, Werner, Dr.rer.nat.**
**Meerfeldstrasse 46**
**D-6800 Mannheim (DE)**
Erfinder: **Schüssler, Rudolf**
**Oberlacherstrasse 20**
**D-6840 Lampertheim (DE)**
Erfinder: **Van Rijckevorsel, Rainer**
**Freiburger Strasse 3**
**D-6831 Brühl (DE)**

EP 0 036 443 B1

Courier Press, Leamington Spa, England.

**Beschreibung**

Die Erfindung betrifft eine Blutlanzettenvorrichtung nach dem Oberbegriff des Anspruchs 1.

Um für Diagnosezwecke eine Entnahme von nur geringen Mengen Blut vorzunehmen, werden Lanzetten verwendet, die von einer Laborhelferin bei dem betreffenden Patienten in die Fingerkuppe oder auch andere Körperteile kurz eingestochen werden. Dieses Verfahren hat den Nachteil, daß der Stichvorgang selbst sichtbar ist und die entstehende Wunde abhängig vom Benutzer der Lanzette und daher nie genau reproduzierbar ist. Außerdem muß eine zweite Person den Stechvorgang vollziehen, da die Hemmschwelle zum Selbstverletzen sehr groß ist.

Für den ärztlichen Einsatz sind auch bereits eine Vielzahl von Lanzettenvorrichtungen vogeschlagen worden, bei denen der Einstichvorgang mechanisiert ist. Dabei wird in der Regel eine Feder benutzt, die den Einstichvorgang antreibt. Um die Einstichtiefe festzulegen, kann dabei am Gehäuse der Lanzettenvorrichtung ein Anschlag vorgesehen sein, gegen den das lanzettenführende Bauelement anschlägt. Dabei entsteht jedoch eine erhebliche Erschütterung, durch die es erheblich erschwert wird, bei jeder Benutzung einen gleichmäßig sauberen Einstich zu erhalten.

In der US—A—3 030 959 ist eine fortgeschrittene Ausführungsform einer ärztlichen Blutlanzettenvorrichtung beschrieben, die außer der Antriebsfeder eine zweite Feder aufweist, durch die der Schwung bei der Einstichbewegung abgebremst wird.

Dabei befinden sich beide Federn in ständiger Wirkverbindung mit der Lanzette. Die Folge davon ist, daß der Einstich zwar weich, jedoch relativ langsam erfolgt, weil beim Abbremsen die kinetische Energie, die durch die antreibende Feder vermittelt wird, nur langsam durch die entgegenwirkende Kraft der Rückführfeder aufgezehrt wird. Für den Patienten ist jedoch ein schneller Einstichvorgang wünschenswert, weil dieser weniger schmerzhaft empfunden wird. Der ständige Eingriff beider Federn hat außerdem zur Folge, daß die Einstichtiefe sehr empfindlich von der Federkraft der Federn abhängt. Aus diesem Grund muß jede derartige Lanzettenvorrichtung individuell justiert werden, wie dies auch bei der US—A—3 030 959 vorgesehen ist. Eine solche Justierung ist jedoch unerwünscht, insbesondere wenn die Lanzettenvorrichtung für die Handhabung durch Laien geeignet sein soll.

Aus der DE—A—2 642 896 ist eine weitere Lanzettenvorrichtung bekannt, die ebenfalls zwei Federn verwendet und sich ebenfalls mit dem Problem von Erschütterungen befasst. Hier wird jedoch ausschließlich der beim Auslösen der Einrichtung entstehende Rückstoß bekämpft, und zwar durch ein geeignetes Ausgleichsgewicht. Der Einstichvorgang wird dagegen in bekannter Weise durch einen Anschlag begrenzt, so daß die dadurch erzeugten Erschütterungen bei dieser bekannten Vorrichtung nicht beseitigt sind.

Der Erfindung liegt die Aufgabe zugrunde, eine Blutlanzettenvorrichtung der eingangs beschriebenen Art dahingehend weiterzubilden, daß sie besser als die bisher bekannten Einrichtungen zur Selbstentnahme von Blut verwendet werden kann. Diese Selbstentnahme von Blut ist insbesondere für die Diabetiker-Selbstkontrolle von großer Bedeutung, weil diese nicht zur zu einer Entlastung des medizinischen Personals führt, sondern eine wesentlich verbesserte Einstellung der medikamentösen Behandlung von Diabetikern ermöglicht. Dadurch können insbesondere schwere Spätschäden der Diabetes vermieden werden.

Insbesondere geht es dabei darum, zugleich einen schnellen Einstich, ein erschütterungsfreies Abbremsen der Einstichbewegung und eine reproduzierbare Einstichtiefe zu erreichen.

Die gestellte Aufgabe wird durch eine Blutlanzettenvorrichtung gemäß Anspruch 1 gelöst.

Der Federantrieb für die Lanzette schließt zwei verschiedene Federn ein. Eine dieser beiden Federn treibt den Einstichvorgang und ist bevorzugt so ausgelegt, daß sie in ihrem Wirkbereich eine stärkere Kraft hat als die zweite Feder, welche zum Antrieb der Rückführbewegung der Lanzette gedacht ist. Durch diese Maßnahme wird erreicht, daß die Einstich- und Rückführbewegung voneinander entkoppelt sind und in ihrer jeweiligen Geschweindigkeit und Stärke den Anforderungen angepaßt werden können. Bevorzugt wirkt beim Einstichvorgang die stärkere Feder gegen die Kraft der schwächeren. Eine geeignete Mechanik, wie sie beispielhaft weiter unten näher beschrieben wird, unterbricht den Eingriff der stärkeren Feder an einem bestimmten Punkt des Lanzettenhubs. Die Lanzette schwingt dann nur noch um ein kurzes durch ihre Trägheit und die Kraft der den Einstichvorgang treibenden Feder bestimmtes Stück weiter und wird anschließend durch die die Rückführbewegung treibende Feder in Richtung auf die Ausgangslage zurückbewegt.

Die zuvor beschriebene Entkopplung der die Einstichbewegung treibenden Feder von der Lanzette läßt sich gemäß einer weiteren bevorzugten Ausführungsform der Erfindung mit Hilfe eines Winkelhebels erreichen, der von dieser Feder angetrieben wird. Ein solcher Winkelhebel ermöglicht zugleich eine kompakte und kostengünstige Bauweise der Einrichtung.

Das Auslöseelement ist bevorzugt im Bereich der Austrittsöffnung angeordnet. Dadurch wird erreicht, daß die Einstichbewegung der Lanzettenvorrichtung durch das Aufdrücken derselben auf das Körperteil, welchem Blut zu entnehmen ist, ausgelöst wird. Dies hat den wesentlichen Vorteil, daß der Benutzer die Blutlanzettenvorrichtung nicht mehr während des Auslösens zurückziehen kann. Es wird durch diese Maßnahme sogar ein gleichmäßiges Andrücken der Einrichtung, beispielsweise auf die Fingerbeere, gewährleistet. Dies ist von großer Bedeutung, um reproduzierbar eine möglichst optimale Wunde zu erreichen. Bei den bisherigen

Lanzettenvorrichtungen könnte es dagegen leicht passieren, daß durch krampfhaft festes Andrücken der Vorrichtung auf die Fingerbeere eine zu tiefe Wunde entstand, welche schlecht verheilt, oder daß die Wunde durch plötzliches Zurückziehen des Gerätes so klein ausfiel, daß nicht genügend Blut gewonnen werden konnte.

Gemäß einer bevorzugten Ausführungsform ist das Auslöseelement als in das Gehäuse eingelassene Auslösetaste ausgebildet, so daß es für den Benutzer im Moment des Einstechens nicht sichtbar ist. Auch durch diese Maßnahme wird die Hemmschwelle bei der Benutzung verringert und dadurch letztlich eine gleichmäßigere Wunde erreicht.

Zum Herstellen der kraftschlüssigen Wirkverbindung zwischen dem Auslöseelement und der Arretierung der Lanzette wird gemäß einer weiteren bevorzugten Ausführungsform der Erfindung die Verwendung eines Schiebers vorgeschlagen, der mit dem Auslöseelement verbunden ist, wobei gleichzeitig das Auslöseelement durch eine Druckfeder nach dem Auslösen in seine Ausgangsposition zurückgedrückt wird. Diese Schieberverbindung weist wenige mechanische Bauteile auf, ist deshalb einfach zu realisieren und zuverlässig in der Funktion. Es sind jedoch zahlreiche weitere Realisierungen der Wirkverbindung zwischen Auslöser und Arretierung denkbar, beispielsweise Hebelverbindungen, Seilzüge oder auch hydraulische Einrichtungen.

Bevorzugt ist in der Lanzettenvorrichtung eine Lanzettenhalterung vorgesehen, in welcher die Lanzette auswechselbar, jedoch im Betrieb in mit der für den Einstichvorgang notwendigen Ortsfestigkeit untergebracht ist. Dadurch kann für jeden Einstichvorgang eine neue Lanzette verwendet werden, so daß das Reinigen und Desinfizieren der Lanzettenspitze entfällt. Um das Auswechseln der Lanzette möglichst einfach zu machen, läßt sich gemäß einer weiteren bevorzugten Ausführungsform der Lanzettenhalter über einen Betätigungshebel zur Aufnahme der Lanzette in Richtung auf die Austrittsöffnung der Lanzette bewegen. Als Betätigungshebel kann vorteilhafterweise die gleiche Einrichtung verwendet werden, die auch zum Spannen der Lanzettenvorrichtung dient.

Weitere bevorzugte Ausführungsformen dienen insgesamt dem Zweck, das Auswechseln der Lanzette zu einem Vorgang zu machen, der sich ohne Zutun des Benutzers zwischen jeder Benutzung der Lanzettenvorrichtung automatisch abspielt. Zu diesem Zweck ist eine Auswurfautomatik mit Wiederholsperre vorgesehen, die bevorzugt in Form eines mit der Halteeinrichtung für die Lanzette in dem Lanzettenhalter verbundenen Klinkenhebels realisiert ist. Dieser Klinkenhebel weist einerseits eine Klinke auf, die formschlüssig mit einem entsprechenden Teil der Lanzette zusammenwirkt. Auf der anderen Seite wirkt der Klinkenhebel mit einem Widerlager zusammen, das sich ortsfest in dem Gehäuse der Lanzettenvorrichtung befindet. Wird nun die Halteeinrichtung der Lanzette zur Aufnahme einer neuen Lanzette vom Benutzer über eine geeignete Hebeleinrichtung beziehungsweise einen Bedienungsknopf auf die Lanzettenaustrittsöffnung zu bewegt, so stößt das von der Klinke abgewandte Ende des Klinkenhebels an das Widerlager, wodurch die Lanzette zwangsweise ausgeworfen wird. Um diesen Auswurfvorgang zu erleichtern, ist in der Lanzettenhalteeinrichtung ein bewegliches Gewicht vorgesehen, das auf der in die Halteeinrichtung eingeführten Lanzette aufliegt. Durch die Massenträgheit dieses Gewichtes wird das Auswerfen der Lanzette in dem Moment, in dem der Klinkenhebel an das Widerlager stößt, erleichtert.

Nach dem Auswerfen der benutzten Lanzette wird der Klinkenhebel gemäß einer weiter bevorzugten Ausführungsform durch eine geeignete Feder in eine solche Position gebracht, in der sein Betätigungsarm nicht mehr im Wirkbereich des Widerlagers liegt, wenn der Lanzettenhalter in Richtung auf die Austrittsöffnung der Vorrichtung bewegt wird, um die neue Lanzette aufzunehmen. Durch das Einführen der neuen Lanzette wird der Klinkenhebel gegen die Kraft der erwähten Feder in eine Position zurückgedrückt, in der sein Betätigungsarm wieder im Wirkbereich des Widerlagers liegt. Gemäß einer weiteren bevorzugten Ausführungsform der Erfindung sind nun der Angriffspunkt des Widerlagers am Betätigungsarm, die Lagerachse des Klinkenhebels und die Klinke relativ zu einander so angeordnet, daß bei der Bewegung des Lanzettenhalters in Rückführrichtung das Widerlager auf den Klinkenhebel eine Kraft in Schließrichtung der Klinke ausübt. Der Klinkenhebel und/oder da Widerlager sind geringfügig elastisch ausgeführt oder gelagert, um das Rückführen des Lanzettenhalters mit der eingeführten Lanzette zu ermöglichen. Bevorzugt ist der Klinkenhebel U-förmig ausgebildet.

Durch die Kombination der zuletzt genannten Maßnahmen wird erreicht, daß die Lanzette nach der Benutzung zwangsweise ausgeworfen wird und anschließend eine neue Lanzette auf einfache Weise eingeführt werden kann. Eine wiederholte Benutzung einer Lanzette mit der daraus resultierenden erheblichen Infektionsgefahr wird dadurch zuverlässig vermieden.

Anhand mehrerer in den Figuren dargestellter Ausführungsbeispiele wird die Erfindung nachstehend näher erläutert. Es zeigt:

Fig. 1a eine erfindungsgemäße Blutlanzettenvorrichtung in der Seitenansicht mit abgenommenem Gehäusedeckel im entspannten Zustand,

Fig. 1b die Blutlanzettenvorrichtung gemäß Fig. 1a im gespannten und arretierten Zustand,

Fig. 2 eine andere Ausführungsform der Blutlanzettenvorrichtung, ebenfalls in der Seitenansicht mit abgenommenem Gehäusedeckel,

Fig. 3 eine dritte Ausführungsform einer erfindungsgemäßen Blutlanzettenvorrichtung in einer Ansicht wie bei den vorhergehenden Figuren, aber mit teilweise weggeschnittenen Teilen im gespannten und arretierten Zustand,

Fig. 4a eine bevorzugte Ausführungsform eines Lanzettenhalters zur Verwendung in einer erfindungsgemäßen Lanzettenvorrichtung in Seitenansicht und teilweise im Schnitt mit eingeführter Lanzette,

Fig. 4b den Lanzettenhalter gemäß Fig. 4a nach dem Auswerfen der Lanzette,

Fig. 5 einen Schnitt durch den Lanzettenhalter gemäß Fig. 4a entlang der Linie V—V und

Fig. 6 einen Schnitt durch den Lanzettenhalter gemäß Fig. 4a entlang der Linie VI—VI.

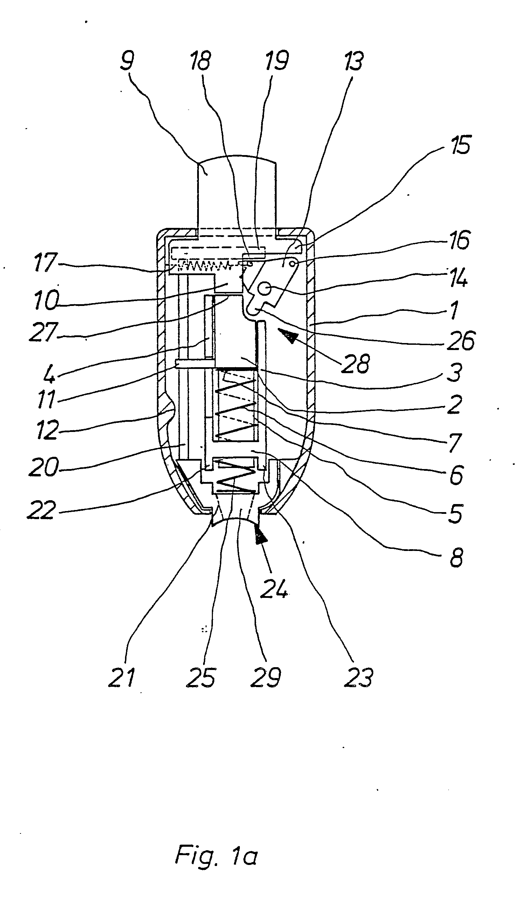

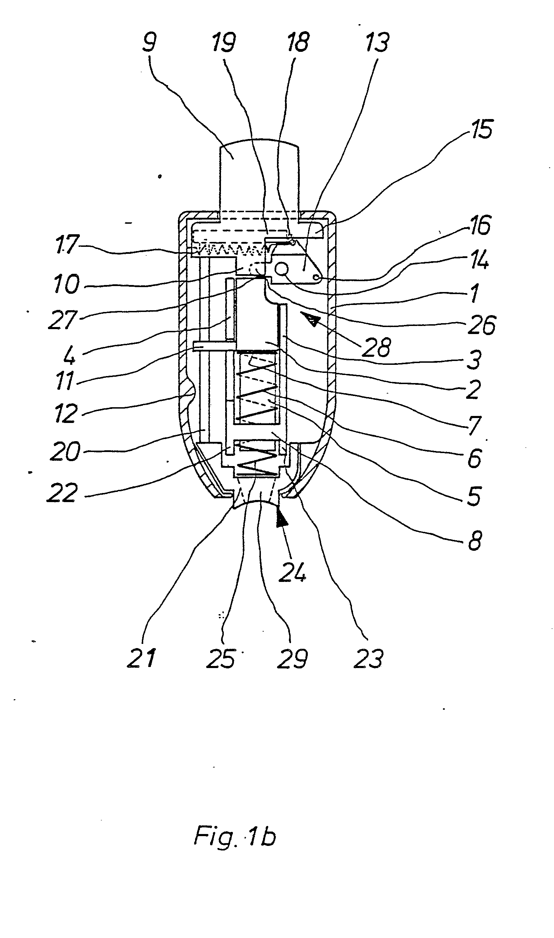

Die in der Fig. 1 gezeigt Blutlanzettenvorrichtung besteht im wesentlichen aus dem Gehäuse 1 und dem darin angeordneten Lanzettenhalter 2. Der Lanzettenhalter 2 ist von den Gleitflächen 3 und 4 seitlich eingefaßt. Die Ober- und Unterseite des Lanzettenhalters 2 wird von den Gehäusewänden geführt. Selbstverständlich sind zahlreiche andere Führungen, die dem Fachmann geläufig sind, zur Führung des Lanzettenhalters zu verwenden. Für die Aufnahme der Lanzette ist im Lanzettenhalter 2 eine Klemmhalterung angeordnet. Das vorstehende untere Ende 5 der Lanzettenhalterung ist zylinderförmig ausgebildet und wird von der Druckfeder 6 umfaßt, die an dem Absatz 7 des Lanzettenhalters 2 und an einer Brücke 8, die am Gehäuse 1 befestigt ist, anliegt. Für die Aufnahme der Lanzette wird der Lanzettenhalter 2 nach unten bewegt und die Lanzette wird in die in der Achse der Lanzettenhalterung liegende Öffnung mit Klemmhalterung eingeführt. Um den Lanzettenhalter 2 entgegen der Federkraft der Feder 6 nach unten bewegen zu können, ist der Betätigungsknopf 9 vorgesehen, der aus dem Gehäuse 1 hervorragt und der über den Steg 10 mit dem Lanzettenhalter 2 verbunden ist. Mit der Klemmhalterung im Lanzettenhalter 2 ist der Hebel 11 verbunden, der während der Bewegung des Lanzettenhalters 2 nach unten an dem von der Gehäusewand hervorstehenden Nocken 12 anschlägt und dadurch einer Schwenkbewegung unterworfen wird. Während dieser Schwenkbewegung wird die Lanzettenklemmhalterung freigegeben und die Lanzette selbst kann unter ihrer eigenen Schwerkraft aus der Blutlanzette herausfallen. Hiernach kann eine neue Lanzette eingesetzt werden, die in der Klemmhalterung fest aufgenommen wird. Der Hebel 11 selbst steht unter Federkraft und wird in seine Ausgangslage durch die Feder zurückgedrückt.

Für die automatische Betätigung der Blutlanzette für den Einstichvorgang ist der Winkelhebel 13 vorgesehen, der um die Achse 14 schwenkbar angeordnet ist. Beim Niederdrücken der Taste 9 greift der Vorsprung 15 der Taste an dem Stift 16 des Winkelhebels 13 an und dreht den Winkelhebel 13 entgegen der Kraft der Feder 17 im Uhrzeigersinn. Die Feder 17 ist mit ihrem einen Ende am Gehäuse und mit ihrem anderen Ende an dem Ausleger 18 des Winkelhebels 13 befestigt. Wenn die Taste 9 voll heruntergedrückt ist, schnappt der Ausleger 18 hinter das Ende des gestrichelt eingezeichneten Querbalkens 19 und arretiert dort. Der Querbalken 19 ist über die Stange 20 mit dem Auslöser 21 direkt verbunden. Der Auslöser 21 wird von den Pfeilern 22 und 23 der Brücke 8 geführt. Er ist in gleicher Richtung wie der Lanzettenhalter 2 bewegbar in der Öffnung 24 des Gehäuses 1 angeordnet. In dem Auslöser befindet sich die gestrichelt dargestellte Austrittsöffnung für die Lanzette. Überdie Feder 25 wird der Auslöser 21 geringfügig aus der Öffnung herausgedrückt. Die gezeichnete Lage zeigt den Auslöser 21 im entspannten, d.h. nicht betätigten Zustand, wobei er geringfügig aus dem Gehäuse 1 hervortritt.

Wenn der Ausleger 18 über den Querbalken 19 arretiert ist, befindet sich der Stößel 26 des Winkelhebels 13 oberhalb der Fläche 27 des Lanzettenhalters 2. Diese Stellung ist in Fig. 1b dargestellt.

Durch Andrücken der Blutlanzettenvorrichtung gegen die Blutentahmestelle wird der Auslöser 21 in das Gehäuse 1 eingedrückt. Damit bewegt sich die Stange 20 und der Querbalken 19 nach oben. Der Ausleger 18 des Winkelhebels 13 wird freigegeben und unter der Krft der Zugfeder 17 wird der Winkelhebel 13 um seine Achse 14 gedreht. Über den stößel 26 drückt der Winkelhebel 13 den Lanzettenhalter 2 in einer plötzlichen Bewegung nach unten. Am Ende dieser Bewegung erfolgt der Einstich der Lanzette. Nach erfolgtem Einstich rutscht der Stößel 26 in die Ausnehmung 28 am Lanzettenhalter für eine Rückwärtsbewegung in seine Ruhestellung frei. Diese Rückwärtsbewegung wird durch die Feder 6 bewirkt. Die Hublänge der Einstichbewegung wird durch die weiter unten beschriebenen Maßnahmen so bemessen, daß sie eine optimale Stichwunde bewirkt. Jedenfalls ist sie so gering, daß der Hebel 11 und der Nocken 12 sich nicht berühren.

Nach diesem Vorgang wird die Taste 9 betätigt und dabei das Gehäuse 1 mit der Öffnung 24 nach unten über eine Auffangschale oder dergleichen gehalten. Bei Erreichen des Nockens 12 durch den Hebel 11 wird die Klemmhalterung geöffnet und die Lanzette kann frei herausfallen. Damit ist der Einstichvorgang und die Herausnahme der Lanzette beendet. Es ist zu beachten, daß während dieses Vorganges die Lanzette von Hand nicht mehr berührt werden braucht.

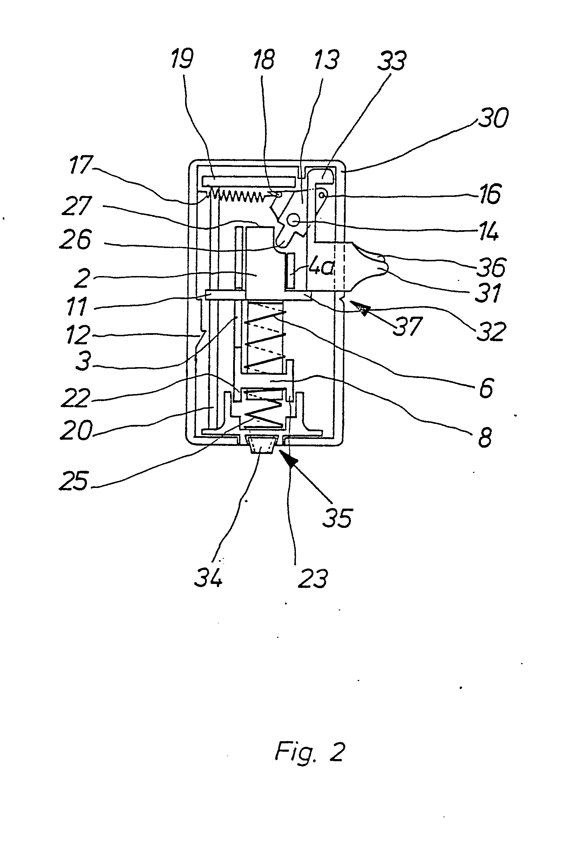

Bei der in der Fig. 2 gezeigten automatischen Blutlanzette ist der prinzipielle Aufbau der Blutlanzette nach Fig. 1 beibehalten worden. Abwandlungen sind jedoch bei der Form des Gehäuses, der Drucktaste und des Auslösers vorgenommen. Bei dieser Blutlanzette wird im Behäuse 30 der Lanzettenhalter 2 über den Verstellhebel 31 für die Aufnahme der Lanzette nach unten bewegt. Der Verstellhebel 31 greift an einem Vorsprung 32 des Lanzettenhalters 2 an. Über den Mitnehmer 33 des Verstellhebels 31 wird während der Bewegung des Verstellhebels 31 nach unten der Stift 16 des Winkelhebels 13 und damit der Winkelhebel 13 selbst mitgenommen und um die Achse 14 gedreht. Der Ausleger 18 kommt hinter dem Ende des Querbalkens 19 zu liegen und wird dadurch arretiert. Der Stößel 26 des Winkelhebels liegt dann an der Fläche 27 des Lanzettenhalters 2 an. Der Lanzettenhalter 2 wird von den Gleitflächen 3 und 4a geführt. Die Druckfeder 6 drückt den Lanzettenhalter 2 in seine gezeichnete Ruhestellung. Die Pfeiler 22 und 23 der Brücke 8 dienen als Gleitflächen für den Auslöser 34, der aus der Öffnung 35 des Gehäses 30 herausragt. Die Druckfeder 25 drückt den Auslöser 34 nach unten. Der Auslöser 34 ist mit dem Querbalken 19 über die Stange 20 verbunden.

Neben dem Verstellhebel 31 ist ein Rastknopf 36 vorgesehen, der bei heruntergedrücktem Verstellhebel 31 in die Nut 37 eingerastet werden kann. Durch diese Maßmahme wird der Lanzettenhalter 2 in seiner unteren Stellung festgestellt, wodurch das Einlegen der Lanzette selbst erleichtert wird. Die Klemmhalterung für die Lanzette hat den Öffnungshebel 11 mit Anschlagnocken 12 wie in der Fig. 1 beschrieben.

Durch eine entsprechende Abstimmung zwischen der Auflagefläche 27 und der Größe des Stößels 26 kann die Hublänge des Lanzettenhalters 2 sehr genau eingestellt werden. In praktischen Versuchen hat sich herausgestellt, daß bei geeigneter Abstimmung der Federn 6 und 17 eine sehr genaue Reproduzierbarkeit der Einstichtiefe zu erreichen ist. Der Einstichvorgang läuft sehr schnell ab, so daß er kaum im einzelnen zu analysieren ist. Jedenfalls wird durch die relative Lage des Stößels 26 und der Fläche 27 und ihre jeweilige Ausdehnung die Hubposition des Lanzettenhalters 2 festgelegt, in welcher der Eingriff zwischen dem Winkelhebel 13 und dem Halter 2 unterbrochen wird. In diesem Moment wirkt die Feder 17 nicht mehr in Einstichrichtung. Der Halter 2 bewegt sich aber aufgrund seiner Trägheit noch um eine bestimmte und, wie die erwähnten praktischen Versuche ergeben haben, genau reproduzierbare Weglänge nach unten gegen die Kraft der Feder 6. Nach dem Umkehrpunkt wird der Halter 2 durch die Feder 6 in seine Ausgangslage zurückgeführt, die in den Fig. 1a und 2 dargestellt ist.

Durch diese Maßnahmen kann die Größe der Stichwunde vorbestimmt werden. Bei jeder Betätigung der Blutlanzette bleibt diese Stichwunde völlig gleichmäßig. Der Nocken 12 in Verbindung mit dem Hebel 11 stellt eine Auswurfmechanik dar, die bei jedem Ladevorgang die Lanzette aus der Klemmhalterung herausfallen laßt.

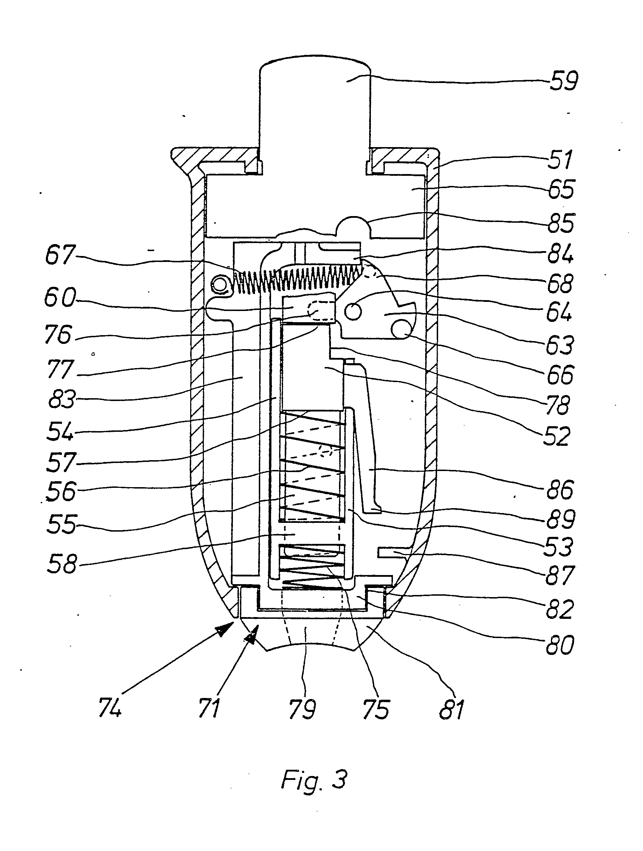

Fig. 3 zeigt eine weitere Ausführungsform der Erfindung, wobei die den zuvor beschriebenen Ausfürungsformen entsprechenden Bauteile mit Bezugszeichen gekennzeichnet sind, die sich aus der Addition der in Fig. 1 für entsprechende Teile verwendeten Bezugszeichen mit der Zahl 50 ergeben.

Man erkennt das Gehäse 51, den Lanzettenhalter 52, die Gleitflächen 53 und 54, das vorstehende Ende 55 des Lanzettenhalters 52, die Druckfeder für die Rückführung 56, die sich auf dem Absatz 57 abstützt, die Brücke 58, den Betätigungsknopf 59 mit seinem Steg 60, den Winkelhebel 63, der sich um eine Achse 64 schwenken läßt und von dem Vorsprung 65 des Betätigungsknopfes 59 über den Stift 66 verschwenkbar ist. Mit dem Bezugszeichen 67 ist die Feder versehen, von der über den Winkelhebel 63 der Einstichvorgang angetrieben wird.

Einige Unterschiede zu den zuvor beschriebenen Figuren bestehen im Aufbau der Bauelemente zum Auslösen des Einstichvorganges. Der in seiner Gesamtheit mit dem Bezugszeichen 71 versehene Auslöser, der in der Gehäuseöffnung 74 liegt, besteht bei der dargestellten Ausführungsform aus zwei Teilen, nämlich einer Auslöserbasis 80 und einem Auslöseaufsatz 81, welcher über eine entsprechende Klemmhalterung 82 mit der Auslöserbasis auswechselbar verbunden werden kann. Beide Teile sind durch die gestrichelt dargestellte Lanzettenaustrittsöffnung 79 durchbrochen und werden zusammen durch die Feder 75 in Richtung auf die Austrittsöffnung belastet. Zu dem Gerät können verschiedene Auslöseraufsätze geliefert werden, die sich in ihrer Dicke geringfügig unterscheiden. Da die Hublänge des Lanzettenhalters und damit der Lanzette relativ zum Gehäuse wie zuvor beschrieben reproduzierbar ist, wird die Einstichtiefe in das gegen den Auslöseraufsatz 81 gedrückte Körperteil umso tiefer, je weniger dick dieser Auslöseraufsatz ausgebildet ist. Durch diese einfache Maßnahme kann in sehr vorteilhafter Weise die Einstichtiefe auf die Haut des einzelnen Patienten abgestimmt und somit dahingehend optimiert werden, daß eine ausreichend ergiebige und zugleich schnell heilende Wunde entsteht.

Weiterhin unterscheidet sich die Ausführungsform nach Fig. 3 dadurch, daß die Verbindungsstange 20 und der Querbalken 19 durch einen einstückig ausgeformten Schieber 83 ersetzt sind, der die Auslösebewegung vom Auslöser 71 zum Winkewhebel 63 überträgt. Dieser ist in der Fig. 3 in der gespannten und arretierten Stellung dargestellt (entsprechend Fig. 1b), in der das Ende 84 des Schiebers 83 mit dem Ausleger 68 des Winkelhebels 63 im Eingriff ist, um diesen zu arretieren. Wird beim Andrücken des Auslösers 71 auf ein entsprechendes Körperteil, welchem Blut entnommen werden soll, der Schieber 83 nach oben bewegt, so wird der Winkelhebel 63 frei und der Stößel 76 schlägt auf die obere Fläche 77 des Lanzettenhalters 52 auf und führt diesen im Einstichvorgang nach unten. Wie zuvor beschrieben, wird der Einstichvorgang dadurch beendet, daß der Stößel 76 in die Ausnehmung 78 des Lanzettenhalters eintritt und damit der Kraftschluß unterbrochen wird. Danach setzt die Rückführbewegung ein, die von der Feder 56 angetrieben wird. Der Betätigungsknopf 58 hat bei der dargestellten Ausführungsform eine Aussparung 85, damit er beim Spannen der Vorrichtung nicht mit der Achse 64 in Berührung kommt. Der Steg 60 des Betätigungsknopfes 59 ist in der Fig. 3 abgeschnitten dargestellt, damit man die Feder 67 und den oberen Teil des Schiebers 83 besser erkennen kann.

Eine weitere Besonderheit der in Fig. 3 dargestellten Ausführungsform ist die darin vorgesehene Auswurfmechanik mit Wiederholsperre. Deren Hauptteile sind der Klinkenhebel 86 und das Widerlager 87.

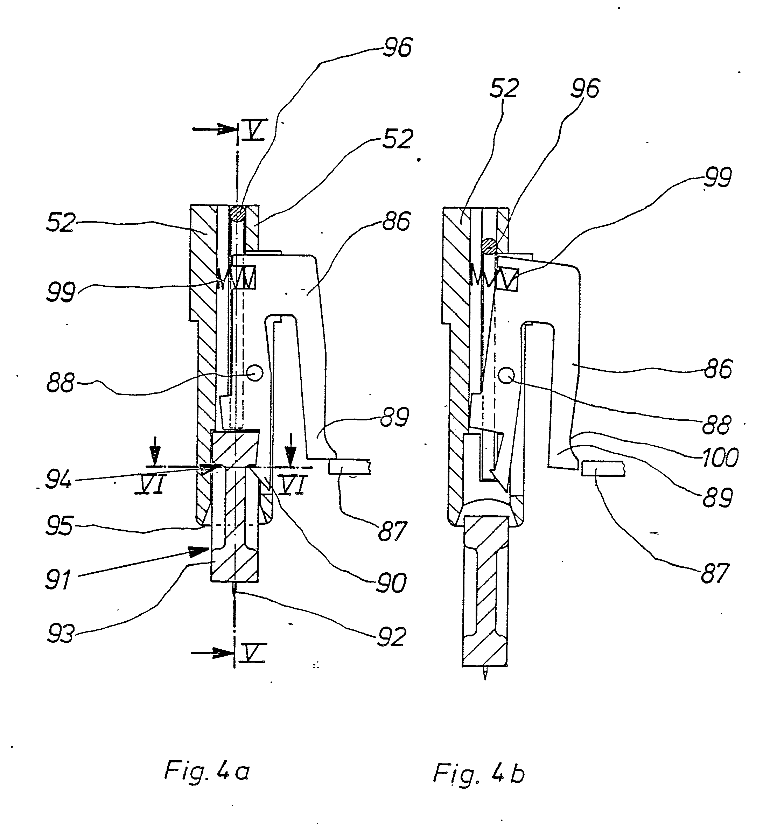

Die Funktion der Auswurfmechanik mit Wiederholsperre ist aus den Fig. 4a, 4b, 5 und 6 deutlicher ersichtlich. Der Klinkenhebel 86 ist um eine Achse 88 schwenkbar und hat einen Betätigungsarm 89 und eine Klinke 90. Er ist in der in den Figuren ersichtlichen Art und Weise in den Lanzettenhalter 52 eingebaut.

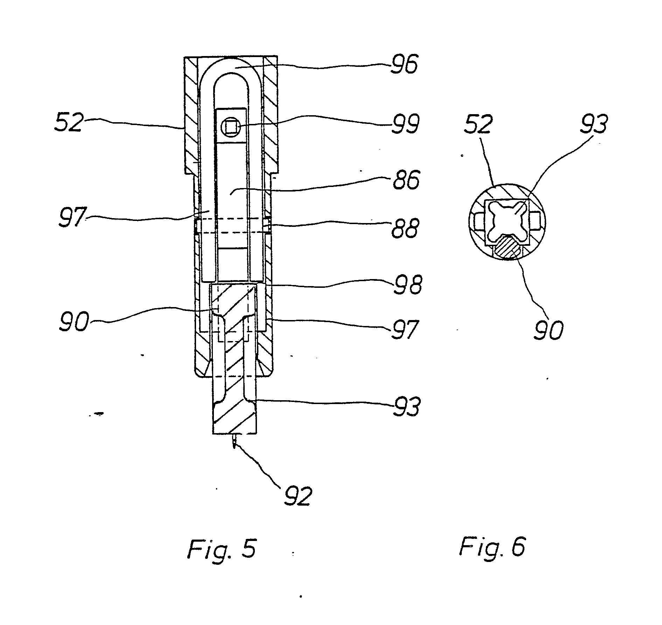

Fig. 4a zeigt den Lanzettenhalter mit eingesetzter Lanzette 91. Die Lanzette 91 besteht in der dargestellten Ausführungsform aus der Lanzettenspitze 92 und einem aus Kunststoff gespritzten Lanzettenkörper 93. Wie aus den Fig. 4a und 6 deutlicher zu ersehen ist, besitzt der Lanzettenkörper Ausnehmungen 94, hinter die die Klinke 90 des Klinkenhebels 86 greift, um die Lanzette 91 zu halten. Aus Fig. 6 ist im Querschnitt zu ersehen, daß die Lanzette im Querschnitt symmetrisch mit vier Ausnehmungen ausgebildet ist, so daß sie vom Benutzer des Gerätes in praktisch beliebiger Lage in den Lanzettenhalter 52 eingeführt werden kann. Zur Fürung und Orientierung der Lanzette weist der Lanzettenhalter 52 schräge Anlaufflächen 95 auf.

In den Figuren erkennt man weiter ein U-förmig ausgeführtes Gewicht 96, welches in vertikaler Richtung in entsprechenden Führungen beweglich ist und auf der Oberkante der Lanzette 91 aufliegt. Schließlich erkennt man noch eine Feder 99, die den Klinkenhebel 86 im Uhrzeigersinn belastet, also in Schließrichtung der Klinke.

Im folgenden wird anhand der Fig. 4 bis 6 der Vorgang des Auswerfens der Lanzette und des Einführens einer neuen Lanzette beschriben. Dieser Vorgang schließt sich jeweils dem zuvor beschriebenen Einstechvorgang an, wobei wesentlich ist, daß der Hub des Lanzettenhalters beim Einstechvorgang so gering ist, daß der Arm 89 des Klinkenhebels 86 dabei nicht mit dem Widerlager 87 in Berührung kommt.

Zum Auswerfen der Lanzette wird über den Betätigungsknopf 59 der Lanzettenhalter nach unten bewegt, bis er in der in Fig. 4a dargestellten Stellung ist. Durch das Widerlager 87 wird der Betätigungsarm 89 beim weiteren Abwärtsdrücken nach rechts außen bewegt, wodurch sich die Klinke 90 ebenfalls nach rechts bewegt und den Lanzettenkörper freigibt. Das Gewicht 96 erhält zusammen mit dem Lanzettenhalter durch das Betätigen des Knopfes 59 einen Impuls in Abwärtsrichtung. Wenn diese Abwärtsrichtung dann durch das Anschlagen des Betätigungsarmes 89 an dem Widerlager 87 unterbgochen wird, übt das Gewicht aufgrund seiner Trägheit, da es in senkrechter Richtung beweglich gelagert ist, einen Impuls auf den Lanzettenhalter aus. Dadurch ist es möglich, daß auch verhältnismäßig leicht gebaute Lanzetten, die entsprechend kostengünstig herzustellen sind, zuverlässig ausgeworfen werden.

Wenn die Lanzette 91 ausgeworfen ist, bewegt sich der Klinkenhebel 86, getrieben durch die Feder 99, im Uhrzeigersinn und der Betätigungsarm 89 kommt in eine Position, die nicht mehr im Wirkbereich des Widerlagers 87 liegt (Fig. 4b).

Jetzt kann der Lanzettenhalter zur Aufnahme einer neuen Lanzette weiter nach unten bewegt werden. Durch die Öffnung 78 wird nun vom Benutzer eine neue Lanzette von unten eingeführt, wobei der Lanzettenhalter in einer Stellung ist, die tiefer liegt als in Fig. 4b dargestellt ist.

Beim Einführen der Lanzette 91 wird der Klinkenhebel 86 gegen die Federkraft zurückgedreht und die Klinke 90 greift hinter die entsprechende Ausnehmung 94 des Lanzettenkörpers 93 ein. Dabei wird der Betätigungsarm 89 des Klinkenhebels 96 derart nach rechts bewegt, daß die Mulde 100 sich im Bereich des Widerlagers 87 befindet.

Der Klinkenhebel 86 ist, insbesondere im Bereich seines Betätigungsarmes 89 aus elastischem Material hergestellt, so daß es möglich ist, daß bei Loslassen des Betätigungsknopfes 59 der Lanzettenhalter 52 durch die Feder 6 nach oben zurückgetrieben wird, wobei die rechte Außenseite des Klinkenhebels 86 am Widerlager 87 anstößt.

Nachdem der Lanzettenhalter 52 somit in seine Ruhelage zurückgekehrt ist, ist wieder die in Fig. 3 dargestellte Position erreicht, in der ein neuer Einstichvorgang ausgelöst werden kann.

**Patentansprüche**

1. Blutlanzettenvorrichtung zur Entnahme von Blut für Diagnosezwecke mit einem Gehäuse (1, 51) mit einer Austrittsöffnung (29, 79) für die Lanzette, einer Lanzettenführung (3, 4; 53, 54) zur Führung der Einstich- und Rückführbewegung der Lanzette, einem Federantrieb (6, 77; 56, 67) für die Lanzettenbewegung, einer Arretierung (18, 19; 68, 84), durch die die Lanzette in einer von dem Körperteil, welchem Blut zu entnehmen ist, entfernten Position gehalten wird und mit einem Auslöseelement (21, 71), das am Gehäuse angeordnet ist und das mit der Arretierung (18, 19, 84, 68) in Wirkverbindung (20, 83) steht, wobei der Federantrieb zwei Federn einschließt, von denen die erste Feder (17, 67) die Einstichbewegung antreibt und die zweite Feder (6, 56) die Rückführbewegung antreibt, dadurch gekennzeichnet, daß die Kraftübertragung von der ersten Feder (17, 67) auf die Lanzette mittels einer Mechanik erfolgt, die gegen Ende der Einstichbewegung den Kraftschluß zwischen der ersten Feder (17, 67) und der Lanzette unterbricht.

2. Vorrichtung nach Anspruch 1, dadurch gekennzeichnet, daß die Federkräfte der beiden Federn (6, 17; 56, 67) verschieden sind und die Federkraft der zweiten Feder (6, 56) während mindestens eines Teils der Einstichbewegung gleichzeitig mit der Federkraft der ersten Feder (17, 67) auf die Lanzette einwirkt, wobei die Federkraft der zweiten Feder kleiner ist als die der ersten Feder und in entgegengesetzter Richtung wirkt.

3. Vorrichtung nach einem der Ansprüche 1 oder 2, dadurch gekennzeichnet, daß die Mechanik zur Kraftübertragung von der ersten Feder auf die Lanzette einen Winkelhebel (13, 63) einschließt, der um eine Achse (14, 64) drehbar ist, wobei der Winkelhebel (13, 63) einerseits mit einem auf die Lanzette bzw. einen Lanzettenhalter (2, 52) wirkenden Stößel (26, 76) fest verbunden ist und andererseits einen Ausleger (18, 68) aufweist, an dem die Feder (17, 67) angelenkt ist, und wobei der Stößel (26, 76) so angeordnet ist, daß er gegen Ende der Einstichbewegung in eine Ausnehmung (28, 78) der Lanzette bzw. des Lanzettenhalters (2, 52) eingreift, wodurch der Kraftschluß zwischen dem Stößel (26, 76) und der Lanzette unterbrochen wird.

4. Vorrichtung nach einem der Ansprüche 1 bis 3, dadurch gekennzeichnet, daß das Auslöseelement (21, 71) eine die Austrittsöffnung (29, 79) umgebende Auslösetaste (21, 71) ist, die so angeordnet ist, daß sie bei Annäherung an das Körperteil, welchem Blut zu entnehmen ist, für den Benutzer nicht sichtbar ist.

5. Vorrichtung nach Anspruch 4, dadurch gekennzeichnet, daß die Arretierung einen auf den Winkelhebel (13; 63) wirkenden Schieber (19, 20; 83) einschließt, der mit dem aus der Gehäuseöffnung (24, 35; 84) für die Lanzette (2; 52) hervorstehenden Auslöseelement (21, 34; 71) verbunden ist, wobei das Auslöseelement (21, 34; 71) durch eine Druckfeder (25; 75) in seiner Lage gehalten wird.

6. Vorrichtung nach einem der Ansprüche 1 bis 5, dadurch gekennzeichnet, daß die Rückführbewegung der Lanzette in ihre Ruhestellung durch eine sich einerseits an der Lanzette, andererseits am Gehäuse (1; 51) abstützende Druckfeder (6; 56) erfolgt.

7. Vorrichtung nach einem der Ansprüche 1 bis 6, dadurch gekennzeichnet, daß die Lanzette im Betriebszustand fest aber auswechselbar, in einem Lanzettenhalter (2; 52) gelagert ist.

8. Vorrichtung nach einem der Ansprüche 1 bis 7, dadurch gekennzeichnet, daß der Lanzettenhalter (2; 52) über einen Betätigungshebel (9, 31; 59) zur Aufnahme der Lanzette bewegbar ist.

9. Vorrichtung nach einem der Ansprüche 7 oder 8, dadurch gekennzeichnet, daß die Halteeinrichtung für die Lanzette in dem Lanzettenhalter (52) einen um eine Lagerachse (88) schwenkbaren Klinkenhebel (86) mit einer Klinke (90) einschließt, welche mit einer entsprechenden Ausformung der Lanzette (91) diese haltend zusammenwirkt, wobei der Klinkenhebel (86) an seinem vom der Klinke (90) abgewandten Ende einen Betätigungsarm (89) hat, der so angeordnet ist, daß er bei in den Lanzettenhalter (52) eingeführter Lanzette bei Bewegung des Lanzettenhalters im Wirkbereich eines Widerlagers (87) liegt.

10. Vorrichtung nach Anspruch 9, dadurch gekennzeichnet, daß die Lagerachse des Klinkenhebels (86), die Klinke (90) und der Angriffspunkt des Widerlagers (87) am Betätigungsarm (89) relativ zueinander so angeordnet sind, daß bei der Bewegung des Lanzettenhalters (52) in Rückführrichtung das Widerlager (87) auf den Klinkenhebel (86) eine Kraft in Schließrichtung der Klinke (90) ausübt.

11. Vorrichtung nach Anspruch 10, dadurch gekennzeichnet, daß der Klinkenhebel (86) im wesentlichen U-förmig ausgebildet ist, wobei sich das U in Einstichrichtung öffnet und der Angriffspunkt des Widerlagers (87) am Betätigungsarm (89) und die Klinke (90) an den beiden Schenkeln des U weiter zu desen Enden hin angeordnet sind, während die Lagerachse (88) näher zu desesn Mitte hin angeordnet ist.

12. Vorrichtung nach einem der Ansprüche 7 bis 11, dadurch gekennzeichnet, daß in der Haltevorrichtung (52) ein relativ zu dieser bewegliches Gewicht (96) oberhalb der Aufnahme für die Lanzette (91) so angeordnet ist, daß es mit der eingeführten Lanzette (91) in Kontakt steht.

**Revendications**

1. Appareil comportant une lancette à saignée pour prises de sang en vue d'un diagnostic, comprenant un boîtier (1, 51) ayant un orifice de sortie (29, 79) pour la lancette, un guidage pour la lancette (3, 4; 53, 54) guidant les mouvements d'incision et de retrait de la lancette, un entraînement à ressort (6, 77; 56, 67) pour le déplacement de la lancette, un dispositif d'arrêt (18, 19; 68, 84) maintenant la lancette en une position éloignée de la partie de corps où doit s'effectuer la prise de sang et un dispositif déclencheur (21, 71) disposé sur le boîtier et relié fonctionnellement (20, 83) au dispositif d'arrêt (18, 19; 84, 68), l'entraînement à ressort comportant deux ressorts, dont le premier ressort (17, 67) engendre le mouvement d'incision et le second ressort (6, 56) engendre le mouvement de retrait, caractérisé en ce que le transfert des forces du premier ressort (17, 67) sur la lancette s'effectue au moyen d'un dispositif mécanique qui interrompt vers la fin du mouvement d'incision la commande par la force entre le premier resort (17, 67) et la lancette.

2. Appareil selon la revendication 1, caractérisé en ce que les forces élastiques des deux ressorts (6, 17; 56, 67) sont différentes et en ce que la force élastique du second ressort (6, 56) agit au moins pendant une partie du mouvement d'incision en même temps que la force élastique du premier ressor (17, 67) sur la lancette, la force élastique du second ressort étant alors inférieure à celle du premier ressort et s'exerçant en sens opposé.

3. Appareil selon l'une des revendications 1 et 2, caractérisé en ce que le dispositif mécanique pour le transfert des forces du premier ressort sur la lancette comporte un levier coudé (13, 63) pouvant pivoter autour d'un axe (14, 64), le levier coudé (13, 63) étant d'une part connecté de façon immobile à un poussoir (26, 76) agissant sur la lancette ou le portelancette et possédant d'autre part un bras (18, 68) auquel est attaché le ressort (17, 67) le poussoir (26, 76) étant disposé de façon à s'engager vers la fin du mouvement d'incision dans une encoche (28, 78) de la lancette ou du porte-lancette (2, 52), ce qui interrompt la commande par la force entre le poussoir (26, 76) et la lancette.

4. Appareil selon l'une des revendication 1 à 3, caractérisé en ce que l'élément déclencheur (21, 71) est une touche de déclenchement (21, 71) entourant l'orifice de sortie (29, 79), cette touche étant disposée de façon qu'elle ne soit pas visible pour l'utilisateur lorsqu'elle s'approche de la partie du corps où doit s'effecteur la prise de sang.

5. Appareil selon la revendication 4, caractérisé en ce que le dispositif d'arrêt comporte un verrou (19, 20; 83) agissant sur le levier coudé (13; 63), ce verrou étant connecté à l'élément déclencheur (21, 34; 71) qui se trouve à l'orifice (24, 35; 84) du boîtier devant la lancette (2; 52), l'élément déclencheur (21, 34; 71) étant maintenu dans sa position au moyen d'un ressort à pression (25; 75).

6. Appareil selon l'une des revendications 1 à 5, caractérisé en ce que le mouvement de retrait de la lancette vers sa position de repos s'effectue au moyen d'un ressort à pression (6; 56) s'appuyant d'une part sur la lancette et d'autre part sur le boîtier (1; 51).

7. Appareil selon l'une des revendications 1 à 6, caractérisé en ce qu'en position de travail la lancette est logée de façon fixe mais échangeable dans un porte-lancette (2; 52).

8. Appareil selon l'une des revendications 1 à 7, caractérisé en ce que le porte-lancette (2; 52) peut être mû au moyen d'un levier d'actionnement (9, 31; 59) pour recevoir la lancette.

9. Appareil selon l'une des revendications 7 et 8, caractérisé en ce que le dispositif de maintien de la lancette dans le porte-lancette (52) comporte un levier (86) à cliquet pourvu d'un cliquet (90) pivotant autour d'un axe (88) de support, coopérant en la bloquant avec une encoche de forme correspondante dans la lancette (91), le levier (86) à cliquet comportant à son extrémité opposée au cliquet (90) un bras d'actionnement (89), disposé de telle façon que, dans le cas où une lancette est introduite dans le porte-lancette, il se trouve dans le rayon d'action d'une butée (87) lors du mouvement du porte-lancette (52).

10. Appareil selon la revendication 9, caractérisé en ce que l'axe de support du levier (86) à cliquet, le cliquet (90) et le point d'attaque de la butée (87) sont disposés sur le bras d'actionnement (89) à des distances relatives entre eux de telle façon qu'en cas d'un mouvement du porte-lancette (52) dans le sens du retrait, la butée (87) exerce sur le levier (86) à cliquet une force dans le sens de la fermeture du cliquet (90).

11. Appareil selon la revendication 10, caractérisé en ce que le levier (86) à cliquet est essentiellement en forme de U, le U s'ouvrant dans le sens de l'incision et le point d'attaque de la butée (87) contre le bras d'actionnement (89) et le cliquet (90) sur les jambages du U étant disposés plus près des extrémités de ceux-ci, tandis que l'axe de support (88) est disposé plus près de leur milieu.

12. Appareil selon l'une des revendications 7 à 11, caractérisé en ce que dans le dispositif d'arrêt (52) est disposé un poids (96) mobile relativement à ce dispositif, au-dessus du point de réception de la lancette (91) de telle façon qu'il soit en contact avec la lancette (91) mise en place.

**Claims**

1. Blood lancet device for the taking of blood for diagnostic purposes with a housing (1, 51) with an exit opening (29, 79) for the lancet, a lancet guide (3, 4; 53, 54) for the guiding of the puncturing and return movement of the lancet, a spring drive (6, 77; 56, 67) for the lancet movement, a stop (18, 19; 68, 84) by means of which the lancet is held in a position remote from the body part from which blood is to be taken and with a release element (21, 71) which is arranged in the housing and which stands in operative connection (20, 83) with the stop (19, 19; 84, 68), whereby the spring drive includes two springs of which the first spring (17, 67) drives the puncturing movement and the second-spring (6, 56) drives the return movement, characterised in that the force transmission from the first spring (17, 67) to the lancet takes place by means of a mechanism which, towards the end of the puncturing movement, interrupts the force connection between the first spring (17, 67) and the lancet.

2. Device according to claim 1, characterised in that the spring forces of the two springs (6, 17; 56, 67) are different and the spring force of the second spring (6, 56) acts during at least a part of the puncturing movement simultaneously with the spring force of the first spring (17, 67) on the lancet, whereby the spring force of the second spring is smaller than that of the first spring and acts in the opposite direction.

3. Device according to one of claims 1 or 2, characterised in that the mechanism for the force transmission from the first spring to the lancet includes an angle lever (13, 63) which is tiltable about an axis (14, 64), whereby the angle lever (13, 63) is, on the one hand, firmly fixed with a striker (26, 76) acting on the lancet or a lancet holder (2, 52) and, on the other hand, has an arm (18, 68) on which the spring (17, 67) is articulated and whereby the striker (26, 76) is so arranged that, towards the end of the puncturing movement, it engages in a recess (28, 78) of the lancet or of the lancet holder (2, 52), whereby the force connection between the striker (26, 76) and the lancet is interrupted.

4. Device according to one of claims 1 to 3, characterised in that the release element (21, 71) is a release element (21, 71) surrounding the exit opening (29, 79) which is so arranged that, in the case of coming close to the body part from which blood is to be taken, it is not visible to the user.

5. Device according to claim 4, characterised in that the stop includes a pusher (19, 20; 83) acting on the angle lever (13; 63) which is connected with the projecting release element (21, 34; 71) for the lancet (2; 52) on the housing opening (24, 35; 84), whereby the release element (21, 34; 71) is held in its position by a pressure spring (25, 75).

6. Device according to one of claims 1 to 5, characterised in that the return movement of the lancet into its resting position takes place by a pressure spring (61; 56) supported, on the one hand, on the lancet, and on the other hand on the housing (1; 51).

7. Device according to one of claims 1 to 6, characterised in that the lancet in the operational state is mounted firmly but exchangeably in a lancet holder (2; 52).

8. Device according to one of claims 1 to 7, characterised in that the lancet holder (2; 52) is movable via an operating lever (9, 31; 59) for the reception of the lancet.

9. Device according to one of claims 7 or 8, characterised in that the holding means for the lancet in the lancet holder (52) includes a ratchet lever (86) with a ratchet (90) tiltable about a mounting axis (88) which, with a corresponding recess of the lancet (91), holdingly cooperates with this, whereby the ratchet lever (86) has, on its end remote from the ratchet (90), an operating arm (89) which is so arranged that, in the case of a lancet inserted into the lancet holder (52), in the case of movement of the lancet holder it lies in the working range of an abutment (87).

10. Device according to claim 9, characterised in that the mounting axis of the ratchet lever (86), the ratchet (90) and the point of engagement of the abutment (87) on the operating arm (89) are so arranged relative to one another that, in the case of the movement of the lancet holder (52) in the return direction, the abutment (87) exerts on the ratchet lever (86) a force in the closing direction of the ratchet (90).

11. Device according to claim 10, characterised in that the ratchet lever (86) is formed substantially U-shaped, whereby the U opens in the puncturing direction and the point of engagement of the abutment (87) on the operating arm (89) and the ratchet (90) are arranged on the two limbs of the U towards its ends, whereas the mounting axis (88) is arranged closer to its middle.

12. Device according to one of claims 7 to 11, characterised in that in the holding device (52) is arranged a weight (96), movable relative to this, above the reception for the lancet (91) in such a manner that it stands in contact with the inserted lancet (91).

*Fig. 1a*

Fig. 1b

19    18    13    33

30

17

27

26

2

11

12

3

22

20

25

16

14

36

31

37    32

6

8

4a

35

34    23

Fig. 2

Fig. 3

Fig. 4a          Fig. 4b

52

96

99

86

97

88

98

97

90

93

92

**Fig. 5**

52

93

90

**Fig. 6**